(19) Europäisches Patentamt
European Patent Office
Office européen des brevets

(11) **EP 1 813 187 B1**

(12) **EUROPEAN PATENT SPECIFICATION**

(45) Date of publication and mention
of the grant of the patent:
**29.10.2008 Bulletin 2008/44**

(51) Int Cl.:
*A61B 5/0205* (2006.01)    *A61B 5/0215* (2006.01)
*A61B 5/029* (2006.01)

(21) Application number: **07001902.1**

(22) Date of filing: **29.01.2007**

(54) **Apparatus for evaluating a patient's hemodynamic status using heart-lung interaction**

Vorrichtung zur Evaluierung des hämodynamischen Zustandes einer Person mittels Herz-Lunge-Interaktion

Appareil pour évaluer l'état hémodynamique d'un patient en utilisant l'interaction coeur-poumon

(84) Designated Contracting States:
**AT BE BG CH CY CZ DE DK EE ES FI FR GB GR HU IE IS IT LI LT LU LV MC NL PL PT RO SE SI SK TR**

(30) Priority: **31.01.2006 DE 102006004415**

(43) Date of publication of application:
**01.08.2007 Bulletin 2007/31**

(73) Proprietor: **IPRM Intellectual Property Rights Management AG**
**6304 Zug (CH)**

(72) Inventors:
• **Michard, Frederic, Dr.**
**91570 Biévres (FR)**
• **Knoll, Reinhold**
**81543 München (DE)**
• **Pfeiffer, Ulrich, Dr.**
**81667 München (DE)**

(74) Representative: **DTS Zürich**
**Resirain 1**
**8125 Zollikerberg/Zürich (CH)**

(56) References cited:
**EP-A- 1 151 719**         **WO-A2-01/70303**
**WO-A2-03/077854**      **US-A- 5 769 082**
**US-A1- 2001 053 881**

## Description

Field of the Invention

The invention relates to an apparatus for evaluating a patient's hemodynamic status using heart-lung interaction induced hemodynamic analysis.

Background of the Invention

It is generally known that for the healthcare management of patients undergoing surgery or who are critically ill clinical strategies are applicable. In particular, patients submitted to e.g. mechanical ventilation require reliable monitoring of their state of health for diagnostics or for deducing therapeutic measures.

It is an important goal of the healthcare management to maintain or improve organs' perfusion. Therefore, it is frequently appropriate to increase the patient's cardiac output CO by means of fluid therapy. The beneficial effect of fluid therapy is observed in approximately 50% of the patients, in the rest fluid therapy may be contraindicated because CO is either sufficient or, in case it is too low, it should primarily be increased with positive inotropic or vasoactive substances only.

For guiding fluid therapy the usage of the respiratory variation in hemodynamic variables is conventional, e.g. in arterial pressure, cardiac stroke volume, pulse-oximetric plethysmographic waveform and pre-ejection period induced by mechanical ventilation. It is generally known that patients with significant respiratory variations in any of the above mentioned hemodynamic variables during mechanical ventilation are able to significantly improve their cardiac output (CO) in response to fluid therapy. Therefore, in order to identify whether a patient is able to benefit from the fluid therapy the patient's respiratory variations are observed.

For assessing the patient's respiratory variations a contour analysis thereof is performed using hemodynamic parameters. Known hemodynamic parameters are, e.g., pulse pressure variation and stroke volume variation, but also pulse-oximetric plethysmographic waveform variations and pre-ejection period variations

These parameters are appropriate for predicting the volume responsiveness and CO response.

From US 6,585,658 and US 5,769,082 methods are known for assessing the respiratory variation in arterial pressure during mechanical ventilation. By using these methods the effects of mechanical ventilation on arterial systolic pressure can be quantified. However, for performing these methods, a manipulation of ventilatory settings is required, either to induce apnea according to US 6,585,658 or to apply incremental and standardized levels of the airway pressure according to US 5,769,082.

It is an object of the invention to provide an apparatus for evaluating e.g. a mechanically ventilated patient's hemodynamic status, wherein the evaluation result is accurate, reliable and simple to achieve.

Summary of the Invention

According to the invention, this object is achieved by an apparatus for evaluating a mechanically ventilated patient's hemodynamic status, adapted to provide a respiratory variation diagram of a hemodynamic variable, and being capable of deriving the value of a hemodynamic parameter for each mechanical breath cycle as well as an assessment of its suitability for the hemodynamic analysis on basis of the respiratory variation diagram.

Due to the fact that according to the invention for each value of the hemodynamic parameter an assessment of its suitability for the hemodynamic analysis is performed, an identified non-suitable value of the hemodynamic parameter is not used for the hemodynamic analysis. Therefore, the results derived from the hemodynamic analysis are generated with suitable values of the hemodynamic parameter only. Hence, the results derived from the hemodynamic analysis are accurate and reliable.

Non-suitable values of the hemodynamic parameter occur in specific clinical situations, during which the quantification of the effects of mechanical ventilation on hemodynamic variables is not clinically relevant or even dangerous. These situations are, e.g., when the patient suffers from cardiac arrhythmia, or when the patient has irregular breathing patterns, i.e. an irregular respiratory frequency or an irregular tidal volume.

Further, since the hemodynamic parameter as well as the suitability assessment thereof is derived from the respiratory variation diagram, a manipulation of ventilatory settings is not required. Therefore, with the inventive apparatus the evaluation result is simple to achieve.

Preferably the hemodynamic variable is the arterial pulse pressure *PP* (the difference between the systolic and the preceding diastolic pressure) and the hemodynamic parameter is the arterial pulse pressure variation *PPV*.

Using the arterial pulse pressure *PP* for quantifying the effects of mechanical ventilation is informative to guide fluid therapy. Therefore, the evaluation result achieved by means of the inventive apparatus is accurate and reliable.

Further, according to a preferred embodiment of the invention, the apparatus comprises any arterial catheter for measuring the arterial pulse pressure (*PP*).

It is preferred that the apparatus is adapted to make use of the equation

$$PPV = 2\frac{PP\max - PP\min}{PP\max + PP\min},$$

wherein *PP*max is the maximum arterial pulse pressure *PP* per mechanical breath cycle, and *PP*min is the minimum arterial pulse pressure *PP* per mechanical breath cycle (PPV is usually expressed as a percentage, therefore 200 may replace 2 in the above equation).

According to an alternative preferred embodiment of the invention, the hemodynamic variable is the arterial systolic pressure and the hemodynamic parameter is the arterial systolic pressure variation, wherein the apparatus preferably comprises an arterial catheter for measuring the arterial systolic pressure.

According to another alternative preferred embodiment of the invention, the hemodynamic variable is the cardiac stroke volume and the hemodynamic parameter is the cardiac stroke volume variation, wherein the apparatus preferably comprises or is connected to an equipment allowing the beat by beat measurement of the cardiac stroke volume.

According to a further alternative preferred embodiment of the invention, the hemodynamic variable is the pulse oximetry plethysmographic waveform and the hemodynamic parameter is the pulse oximetry plethysmographic waveform variation, wherein the apparatus preferably comprises or is connected to a pulse oximeter probe for measuring the pulse oximetry plethysmographic waveform.

According to another alternative preferred embodiment of the invention, the hemodynamic variable is the pre-ejection period and the hemodynamic parameter is the pre-ejection period variation, wherein the apparatus preferably comprises means for simultaneously recording the ECG and either a pulse oximeter plethysmographic signal or an arterial pressure signal for determining the pre-ejection period. The pre-ejection period is defined by Bendjelid, J Appl Physiol (2004)96:337-342.

It is preferred that the apparatus is adapted to perform for each value of the hemodynamic parameter the assessment of the suitability thereof on basis of the detection of cardiac arrhythmia of the patient.

The apparatus is preferably adapted to detect cardiac arrhythmia of the patient by registering the time intervals between the beat-to-beat peaks of the hemodynamic variable, determining a mean time interval value on basis of the respiratory variation diagram, and detecting a mechanical breath cycle comprising at least one time interval exceeding a predetermined deviation from the mean time interval value in order to exclude the value of the hemodynamic parameter assigned to said mechanical breath cycle from the hemodynamic analysis.

The preferred predetermined deviation is 15% of the mean time interval value.

As an alternative, the apparatus is preferably adapted to detect cardiac arrhythmia of the patient by making use of an ECG.

Alternatively, it is preferred that the apparatus is adapted to register time intervals *t* between the beat-to-beat peaks of the arterial pulse pressure *PP*, determine a mean time interval value $\bar{t}$ on basis of the respiratory variation diagram, and wherein the hemodynamic variable is the normalized pulse pressure *PPn* defined as

$$PPn = PP\frac{t}{\bar{t}},$$

and the hemodynamic parameter is the arterial pulse pressure variation *PPV.*

The method is further refined using the normalized pulse pressure *PPn* for calculating the arterial pulse pressure variation *PPV,* since values of the arterial pulse pressure variation *PPV* are even appropriate for hemodynamic analysis, when are extra systolic beats or other irregular heart beat patterns occur

Preferably the apparatus comprises any arterial catheter for measuring the arterial pulse pressure (*PP*).

According to a preferred embodiment of the invention the apparatus is adapted to make use of the equation

$$PPV = 2\frac{PPn\max - PPn\min}{PPn\max + PPn\min},$$

wherein *PPn*max is the maximum normalized arterial pulse pressure (*PPn*) per mechanical breath cycle, and *PPn*min is the minimum normalized arterial pulse pressure (*PPn*) per mechanical breath cycle (30). Because mean time interval for normalization is the same within in this formula. Mean time interval cancels out and could be replaced here by a constant e.g. 1.

Further, it is preferred that the apparatus is adapted to perform for each value of the hemodynamic parameter the assessment of the suitability thereof on basis of the detection of irregular breathing patterns of the patient.

Preferably the apparatus is adapted to detect irregular breathing patterns of the patient by registering the values of the hemodynamic parameter, and detecting at least one mechanical breath cycle pattern comprised of at least three consecutive mechanical breath cycles comprising the values of the hemodynamic parameter exceeding a predetermined deviation from each other in order to exclude the values of the hemodynamic parameter assigned to said mechanical breath cycle pattern from the hemodynamic analysis.

The preferred predetermined deviation is 15% of the mean value of the values of the hemodynamic parameter assigned to said mechanical breath cycle pattern.

As alternatives, it is preferred that the apparatus is adapted to detect irregular breathing patterns of the patient by making use of an airway pressure curve or an airway flow curve, or a central venous pressure curve or a capnographic curve.

As alternatives, it is preferred that the apparatus is adapted to detect irregular breathing patterns by tracking changes of chest dimensions in using either a thoracic bioimpedance signalor a respiratory inductive plethysmographic signal or a magnetometer system signal.

According to a preferred embodiment of the invention, the apparatus is adapted to display the respiratory variation diagram of the hemodynamic variable in such manner that the respiratory variation diagram is shown as vertical bar graph, wherein for each beat-to-beat hemodynamic variable an individual bar is plotted, which e.g. in case of pulse pressure could be defined between the diastolic and systolic pressure value for each beat.

Brief Description of the Drawings

In the following the invention is explained on the basis of a preferred embodiment with reference to the drawings. In the drawings:

Fig. 1 shows an embodiment of an apparatus according to the invention,

Fig. 2 shows four respiratory variation diagrams according to the invention,

Fig. 3 shows eight respiratory variation diagrams according to the invention,

Fig. 4 shows four respiratory variation diagrams, two of them including cardiac arrhythmia indications according to the invention,

Fig. 5 shows four respiratory variation diagrams including irregular breathing pattern indications according to the invention, and

Fig. 6 shows an alternative embodiment of an apparatus according to the invention.

Detailed Description of a preferred Embodiment of the Invention

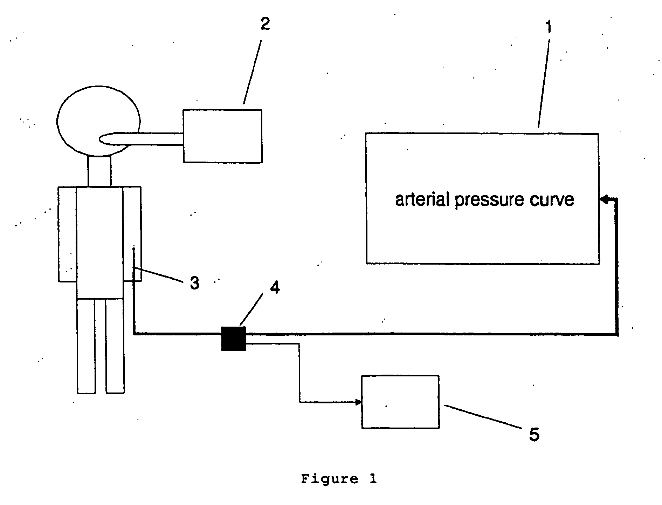

**Figure 1** shows a patient under mechanical ventilation, wherein the patient is ventilated by a ventilator 2 and instrumented with a basic configuration.

The basic configuration comprises an arterial pressure transducer 4 connected via a catheter to an arterial line 3 of the patient. The arterial pressure transducer 4 sends measurement signals to a bedside monitor 5 as well as to an apparatus 1 according to the invention. The signals represent the arterial pulse pressure *PP* measured in line 3.

The apparatus 1 continuously receives the arterial pulse pressure *PP* signals from the arterial pressure transducer 4, generates a respiratory variation diagram on basis of the arterial pulse pressure *PP* signals, records and analyzes the respiratory variation diagrams continuously for performing a hemodynamic analysis.

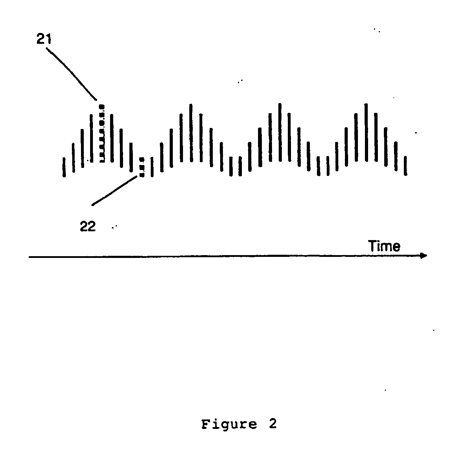

**Figure 2** shows four respiratory variation diagrams on basis of the arterial pulse pressure *PP* signals sent by the arterial pressure transducer 4 to the apparatus 1.

The respiratory variation diagram of the arterial pulse pressure *PP* is shown as vertical bar graph, wherein for each beat-to-beat curve section of the arterial pulse pressure *PP* an individual bar is plotted. EMBEDEach bar represents the arterial pulse pressure *PP* which varies during each mechanical breath between a maximum value *PP*max 21 and a minimum value *PP*min 22.

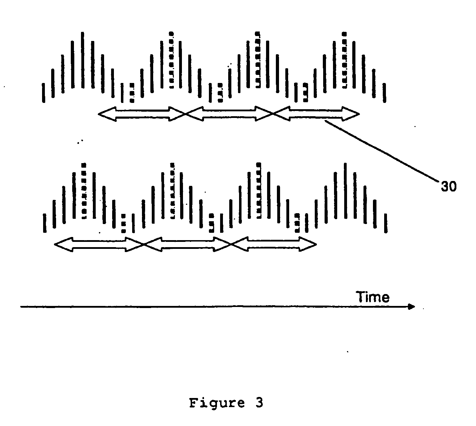

**Figure 3** shows eight respiratory variation diagrams and arrows 30 indicating a single respiratory cycle.

The duration of each respiratory cycle is equal to 60/RF, where RF is the respiratory frequency expressed in 1/min.

The arterial pulse pressure variation *PPV* is calculated over successive respiratory cycles based on the respiratory variation diagram of the arterial pulse pressure *PP* by making use of the equation

$$PPV = 2\frac{PP\max - PP\min}{PP\max + PP\min}.$$

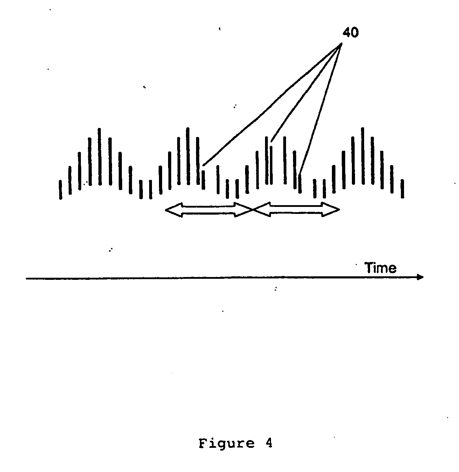

**Figure 4** shows a respiratory variation diagram of the arterial pulse pressure *PP* including cardiac arrhythmia indications 40.

The detection of cardiac arrhythmia is performed by an analysis of the tracing of the arterial pulse pressure *PP* in the respiratory variation diagram. I.e., the time intervals between all peaks (or bars) included in a respiratory cycle are measured. If the variability (defined as standard deviation divided by a mean time interval) of these time intervals is greater than a predetermined threshold value (e.g. 15%), this respiratory cycle is excluded from further hemodynamic analysis.

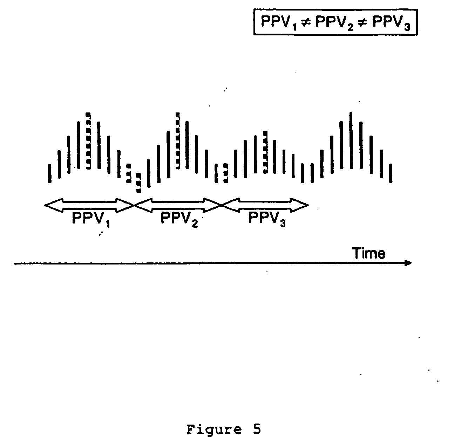

**Figure 5** shows four respiratory variation diagrams of the arterial pulse pressure *PP* in case of irregular breathing pattern (caused by an irregular tidal volume). PPmax and PPmin vary from one respiratory variation diagram to the other, so does PPV.

The detection of cardiac arrhythmia is performed by an analysis of the tracing of the arterial pulse pressure *PP* in the respiratory variation diagram, as illustrated in figure 4. The arterial pulse pressure variation *PPV* is calculated for each respiratory cycle without cardiac arrhythmia. If the variability (defined as standard deviation divided by a mean value of the arterial pulse pressure variation *PPV*) of at least three consecutive *PPV* values is greater than a predetermined threshold value (e.g. 15%), the corresponding *PPV* values will be considered as being invalid and are excluded from the hemodynamic analysis.

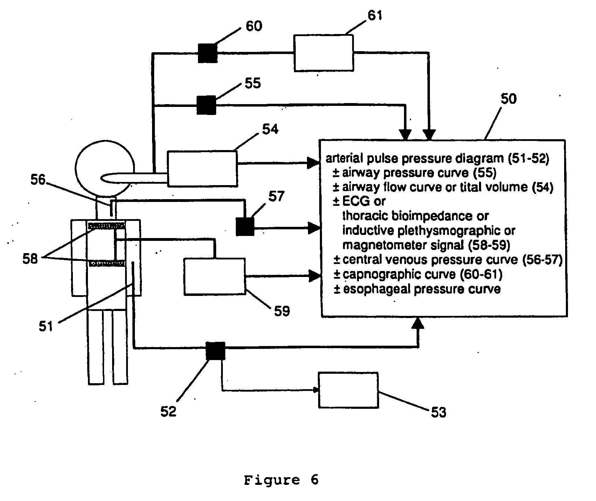

**Figure 6** shows a patient under mechanical ventilation, wherein the patient is ventilated by a ventilator 54 and instrumented with an alternative configuration.

The alternative configuration comprises an arterial pressure transducer 52 connected via a catheter to an arterial line 51 of the patient. The arterial pressure transducer 52 sends measurement signals to a regular, standard bedside monitor 53 as well as to an apparatus 50 according to the invention. The signals represent the arterial pulse pressure PP measured in line 51.

Further, the alternative configuration comprises a central venous pressure transducer 57 connected via a catheter to a central venous line 56 of the patient. The central venous pressure transducer 57 sends measurement signals to the apparatus 50.

Additionally, the alternative configuration comprises an airway pressure transducer 55 connected via the respiratory circuit to the patient. The airway pressure transducer sends measurement signals to the apparatus 50.

Furthermore, the alternative configuration comprises a ECG monitor or a thoracic bioimpedance monitor or a respiratory inductive plethysmography monitor or a magnetometer monitor 59 connected via electrodes (for ECG and thoracic bioimpedance) or elastic bands (for inductive plethysmography) or magnetometer coils (for the magnetometer system) 58 to the patient. The ECG monitor or the thoracic bioimpedance monitor or the respiratory inductive plethysmography monitor or the magnetometer monitor 59 sends measurement signals to the apparatus 50.

For CAPNOGRAPHIC MEASUREMENTS: additionally, the alternative configuration comprises a CO2 sensor (60) on the respiratory circuit connected to a CO2 monitor (61). The CO2 monitor sends CO2 measurement signals to the apparatus.

In case of DIRECT CONNECTION WITH THE VENTILATOR: Additionally, the alternative configuration comprises a connection between the ventilator and the apparatus. The ventilator sends tidal volume, or/and airway pressure, or/and airway flow measurement signals to the apparatus.

In case of SV measurement by ESOPHAGEAL or TRANSCUTANEOUS DOPPLER: Additionally, the alternative configuration comprises an esophageal or transcutaneous Doppler probe connected to a Doppler monitor. The Doppler monitor sends stroke volume measurement signals to the apparatus.

The apparatus 50 continuously receives the signals from the arterial pressure transducer 52, the central venous pressure transducer 57, the airway pressure transducer 55 and the ECG monitor or thoracic bioimpedance monitor or respiratory inductive plethysmography monitor or magnetometer monitor 59 and the ventilator 54, and the CO2 monitor 61. On basis of these signals the apparatus 50 generates respectively an arterial pressure curve, a CVP curve, an airway pressure curve, , an ECG tracing or a bioimpedance signal or a plethysmographic signal or a magnetometer signal, an airway flow and a tidal volume signals, and a capnographic signal.

The ECG is used for the detection of cardiac arrhythmia according to predefined algorithms; the airway pressure curve or the airway flow curve or the capnographic curve or the central venous pressure curve is used for the automatic detection of respiratory frequency and of irregular breathing pattern, e.g. caused by an irregular respiratory frequency or an irregular tidal volume; the thoracic bioimpedance signal or the respiratory inductive plethysmography signal or the magnetometer signal is used for the automatic detection of the respiratory frequency and irregular breathing patterns and the determination of tidal volume.

Taking above mentioned and described into account, a process for evaluating a mechanically ventilated patient's hemodynamic status comprises the steps:

- Providing any arterial catheter to the patient and measuring the arterial pulse pressure PP with the arterial catheter.

- Providing a respiratory variation diagram of the arterial pulse pressure PP, the respiratory variation diagram comprising a vertical bar graph, wherein for each beat-to-beat curve section of the arterial pulse pressure PP an individual bar is provided,

- Deriving the value of the arterial pulse pressure variation PPV for each mechanical breath cycle from the respiratory variation diagram of the arterial pulse pressure PP making use of the equation

$$PPV = 2\frac{PP\max - PP\min}{PP\max + PP\min},$$

wherein PPmax 21 is the maximum arterial pulse pressure PP per mechanical breath cycle 30, and PPmin 22 is the minimum arterial pulse pressure PP per mechanical breath cycle 30.

- Performing for each value of the arterial pulse pressure variation PPV an assessment of the suitability thereof on basis of the detection of cardiac arrhythmia of the patient by registering the time intervals between the beat-to-beat peaks of the hemodynamic variable, determining a mean time interval value on basis of the respiratory variation diagram, and detecting a mechanical breath cycle comprising at least one time interval exceeding a predetermined deviation from the mean time interval value, preferred are 15% of the mean time interval value, and eliminating the value of the arterial pulse pressure variation PPV assigned to said mechanical breath cycle.

- Performing for each value of the hemodynamic parameter the assessment of the suitability thereof on basis of the detection of irregular breathing patterns of the patient by registering the values of the hemodynamic parameter, and detecting at least one mechanical breath cycle pattern comprised of at least three consecutive mechanical breath cycles comprising the values of the hemodynamic parameter exceeding a predetermined deviation from each other, preferred are 15% of the mean value of the values of the hemodynamic parameter assigned to said mechanical breath cycle pattern, and eliminating the values of the arterial pulse pressure variation *PPV* assigned to said mechanical breath cycle patterns.

• Performing the hemodynamic analysis on basis of the non-eliminated values of the arterial pulse pressure variation *PPV*.

As an alternative to the arterial pulse pressure *PP*, the arterial pulse pressure variation *PPV*, the arterial systolic pressure, the arterial systolic pressure variation, or the cardiac stroke volume, the cardiac stroke volume variation and an equipment allowing the beat by beat measurement of the cardiac stroke volume (e.g. arterial pulse contour analysis monitor or esophageal/transcutaneous Doppler monitor), or the pulse oximetry plethysmographic waveform, the pulse oximetry plethysmographic waveform variation and a pulse oximeter probe, or the pre-ejection period, the pre-ejection period variation and the ECG and either a pulse oximeter plethysmographic signal or an arterial pressure signal for determining the pre-ejection period can be used.

Alternatively an ECG can be used to detect cardiac arrhythmia of the patient.

As an alternative to the arterial pulse pressure *PP* and the arterial pulse pressure variation *PPV*, the normalized pulse pressure (*PPn*) can be used. The further steps have to be carried out, namely

• Registering time intervals (*t*) between the beat-to-beat peaks of the arterial pulse pressure (*PP*), determining a mean time interval value ($\bar{t}$) on basis of the respiratory variation diagram, calculating the normalized pulse pressure (*PPn*) by making use of the equation

$$PPn = PP\frac{t}{\bar{t}} \, ,$$

and calculating the values of the arterial pulse pressure variation *PPV* making use of the equation

$$PPV = 2\frac{PPn\max - PPn\min}{PPn\max + PPn\min} \, ,$$

wherein *PPn*max is the maximum normalized arterial pulse pressure *PPn* per mechanical breath cycle, and *PPn*min is the minimum normalized arterial pulse pressure *PPn* per mechanical breath cycle.

Alternatively an airway pressure curve or a central venous pressure curve or a thoracic bioimpedance signal or a tidal volume signal or an airway flow curve or a capnographic signal or a respiratory inductive plethysmographic signal or a magnetometer signal can be used to detect irregular breathing patterns of the patient.

The above mentioned process steps can be carried out by a computer program comprising appropriate instructions.

**Claims**

1.  Apparatus for evaluating a mechanically ventilated patient's hemodynamic status, adapted to analyse a respiratory variation of a hemodynamic variable, and being adapted to derive the value of a hemodynamic parameter for each mechanical breath cycle **characterized by**
    the apparatus being adapted to derive an assessment of the suitability of said value of the hemodynamic parameter for the hemodynamic analysis on basis of a respiratory variation diagram.

2.  Apparatus according to claim 1, wherein the apparatus is adapted to provide a respiratory variation diagram of a hemodynamic variable.

3.  Apparatus according to claim 1 or claim 2, wherein the hemodynamic variable is the arterial pulse pressure (*PP*) and the hemodynamic parameter is the arterial pulse pressure variation (*PPV*).

4.  Apparatus according to claim 3, wherein the apparatus comprises an arterial catheter for measuring the arterial pulse pressure (*PP*).

5.  Apparatus according to claims 3 or 4, wherein the apparatus is adapted to make use of the equation

$$PPV = 2\frac{PP\max - PP\min}{PP\max + PP\min} \, ,$$

wherein *PP*max (21) is the maximum arterial pulse pressure (*PP*) per mechanical breath cycle (30), and *PP*min (22) is the minimum arterial pulse pressure (*PP*) per mechanical breath cycle (30).

6.  Apparatus according to claim 1 or claim 2, wherein the hemodynamic variable is the arterial systolic pressure and the hemodynamic parameter is the arterial systolic pressure variation.

7.  Apparatus according to claim 6, wherein the apparatus comprises an arterial catheter for measuring the arterial systolic pressure.

8. Apparatus according to claim 1 or claim 2, wherein the hemodynamic variable is the cardiac stroke volume and the hemodynamic parameter is the stroke volume variation.

9. Apparatus according to claim 8, wherein the apparatus comprises an equipment allowing the beat by beat measurement of the cardiac stroke volume.

10. Apparatus according to claim 1 or claim 2, wherein the hemodynamic variable is the pulse oximetry plethysmographic waveform and the hemodynamic parameter is the pulse oximetry plethysmographic waveform variation.

11. Apparatus according to claim 10, wherein the apparatus comprises a pulse oximeter probe for measuring the pulse oximetry plethysmographic waveform.

12. Apparatus according to claim 1 or claim 2, wherein the hemodynamic variable is the pre-ejection period and the hemodynamic parameter is the pre-ejection period variation.

13. Apparatus according to claim 12, wherein the apparatus comprises means for simultaneously recording the ECG and either a pulse oximeter plethysmographic signal or an arterial pressure signal for determining the pre-ejection period.

14. Apparatus according to any of claims 1 to 13, wherein the apparatus is adapted to perform for each value of the hemodynamic parameter the assessment of the suitability thereof on basis of the detection of cardiac arrhythmia of the patient.

15. Apparatus according to claim 14, wherein the apparatus is adapted to detect arrhythmia of the patient by registering the time intervals between the beat-to-beat peaks of the hemodynamic variable, determining a mean time interval value on basis of the respiratory variation diagram, and detecting a mechanical breath cycle comprising at least one time interval exceeding a predetermined deviation from the mean time interval value in order to exclude the value of the hemodynamic parameter assigned to said mechanical breath cycle from the hemodynamic analysis.

16. Apparatus according to claim 15, wherein the predetermined deviation is 15% of the mean time interval value.

17. Apparatus according to claim 14, wherein the apparatus is adapted to detect arrhythmia of the patient by making use of an ECG.

18. Apparatus according to claim 1 or claim 2, wherein the apparatus is adapted to register time intervals ($t$) between the beat-to-beat peaks of the arterial pulse pressure ($PP$), determine a mean time interval value ($\bar{t}$) on basis of the respiratory variation diagram, and wherein the hemodynamic variable is the normalized pulse pressure ($PPn$) defined as

$$PPn = PP\frac{t}{\bar{t}},$$

and the hemodynamic parameter is the arterial pulse pressure variation ($PPV$).

19. Apparatus according to claim 18, wherein the apparatus comprises any arterial catheter for measuring the arterial pulse pressure ($PP$).

20. Apparatus according to claims 18 or 19, wherein the apparatus is adapted to make use of the equation

$$PPV = 2\frac{PPn\max - PPn\min}{PPn\max + PPn\min},$$

wherein $PPn$max is the maximum normalized arterial pulse pressure ($PPn$) per mechanical breath cycle, and $PPn$min is the minimum normalized arterial pulse pressure ($PPn$) per mechanical breath cycle (30).

21. Apparatus according to any of claims 1 to 20, wherein the apparatus is adapted to perform for each value of the hemodynamic parameter the assessment of the suitability thereof on basis of the detection of irregular breathing patterns of the patient.

22. Apparatus according to claim 21, wherein the apparatus is adapted to detect irregular breathing patterns of the patient by registering the values of the hemodynamic parameter, and detecting at least one mechanical breath cycle pattern comprised of at least three consecutive mechanical breath cycles comprising the values of the hemodynamic parameter exceeding a predetermined deviation from each other in order to exclude the values of the hemodynamic parameter assigned to said mechanical breath cycle pattern from the hemodynamic analysis.

23. Apparatus according to claim 22, wherein the predetermined deviation is 15% of the mean value of the values of the hemodynamic parameter assigned to said mechanical breath cycle pattern.

24. Apparatus according to claim 21, wherein the apparatus is adapted to detect irregular breathing patterns of the patient by making use of an airway pressure curve or a central venous pressure curve or a thoracic bioimpedance signal or an airway flow curve, or a capnographic curve, or a respiratory inductive plethysmographic signal or a magnetometer signal or a tidal volume measurement.

25. Apparatus according to any of claims 1 to 24, wherein the apparatus is adapted to display the respiratory variation diagram of the hemodynamic variable in such manner that the respiratory variation diagram is shown as vertical bar graph, wherein for each beat-to-beat curve section of the hemodynamic variable an individual bar is plotted, which is defined between the maximum value and the minimum value of beat-to-beat curve section of the hemodynamic variable.

**Patentansprüche**

1. Vorrichtung zur Beurteilung eines hämodynamischen Status eines künstlich beatmeten Patienten, die dazu ausgelegt ist, eine Respirationsveränderung einer hämodynamischen Variable zu analysieren, und dazu ausgelegt ist, den Wert eines hämodynamischen Parameters für jeden künstlichen Atmungszyklus abzuleiten,
   **dadurch gekennzeichnet, dass**
   die Vorrichtung dazu ausgelegt ist, eine Feststellung der Eignung des Werts des hämodynamischen Parameters für die hämodynamische Analyse auf der Grundlage eines Respirationsveränderungsdiagramms abzuleiten.

2. Vorrichtung nach Anspruch 1, wobei die Vorrichtung dazu ausgelegt ist, ein Respirationsveränderungsdiagramm einer hämodynamischen Variable zur Verfügung zu stellen.

3. Vorrichtung nach Anspruch 1 oder Anspruch 2, wobei die hämodynamische Variable der arterielle Pulsdruck (*PP*) und der hämodynamische Parameter die arterielle Pulsdruckveränderung (*PPV*) ist.

4. Vorrichtung nach Anspruch 3, wobei die Vorrichtung einen arteriellen Katheter zum Messen des arteriellen Pulsdrucks (*PP*) aufweist.

5. Vorrichtung nach den Ansprüchen 3 oder 4, wobei die Vorrichtung dazu ausgelegt ist, die Gleichung

$$PPV = 2\frac{PP\max - PP\min}{PP\max + PP\min}$$

zu nutzen, wobei *PP*max (21) der maximale arterielle Pulsdruck (*PP*) pro künstlichen Atmungszyklus (30) ist, und *PP*min (22) der minimale arterielle Pulsdruck (*PP*) pro künstlichen Atmungszyklus (30) ist.

6. Vorrichtung nach Anspruch 1 oder Anspruch 2, wobei die hämodynamische Variable der arterielle systolische Druck und der hämodynamische Parameter die Veränderung des arteriellen systolischen Drucks ist.

7. Vorrichtung nach Anspruch 6, wobei die Vorrichtung einen arteriellen Katheter zum Messen des arteriellen systolischen Drucks aufweist.

8. Vorrichtung nach Anspruch 1 oder Anspruch 2, wobei die hämodynamische Variable das Herzschlagvolumen und der hämodynamische Parameter die Veränderung des Schlagvolumens ist.

9. Vorrichtung nach Anspruch 8, wobei die Vorrichtung ein Gerät aufweist, das die Messung des Herzschlagvolumens für jeden einzelnen Puls aufweist.

10. Vorrichtung nach Anspruch 1 oder Anspruch 2, wobei die hämodynamische Variable die plethysmographische Wellenform der Pulsoxymetrie und der hämodynamische Parameter die Veränderung der plethysmographischen Wellenform der Pulsoxymetrie ist.

11. Vorrichtung nach Anspruch 10, wobei die Vorrichtung einen Pulsoxymetersensor zum Messen der plethysmographischen Wellenform der Pulsoxymetrie aufweist.

12. Vorrichtung nach Anspruch 1 oder Anspruch 2, wobei die hämodynamische Variable die Zeitspanne vor dem Ausstoßen und der hämodynamische Parameter die Veränderung der Zeitspanne vor dem Ausstoßen ist.

13. Vorrichtung nach Anspruch 12, wobei die Vorrichtung Mittel zum gleichzeitigen Aufzeichnen des EKGs und entweder eines plethysmographischen Signals eines Pulsoximeters oder eines arteriellen Pulssignals zum Bestimmen der Zeitspanne vor dem Ausstoßen aufweist.

14. Vorrichtung nach einem der Ansprüche 1 bis 13, wobei die Vorrichtung dazu ausgelegt ist, für jeden Wert des hämodynamischen Parameters die Feststellung der Eignung hiervon auf der Grundlage der Erfassung einer Herzrhythmusstörung des Patienten durchzuführen.

15. Vorrichtung nach Anspruch 14, wobei die Vorrichtung dazu ausgelegt ist, eine Arrhythmie des Patienten zu erfassen, indem die Zeitintervalle zwischen den Spitzenwerten der hämodynamischen Variablen von einem Puls zum nächsten registriert werden, ein mittlerer Zeitintervallwert auf der Grundlage des Respirationsveränderungsdiagramms bestimmt wird, und ein künstlicher Atmungszyklus erfasst wird, der zumindest ein Zeitintervall umfasst, das eine vorbestimmte Abweichung vom mittleren Zeitintervallwert überschreitet, um von der hämodynamischen Analyse den Wert des hämodynamischen Parameters auszuschließen, der dem künstlichen Atmungszyklus zugeordnet ist.

16. Vorrichtung nach Anspruch 15, wobei die vorbestimmte Abweichung 15% des mittleren Zeitintervallwerts beträgt.

17. Vorrichtung nach Anspruch 14, wobei die Vorrichtung dazu ausgelegt ist, eine Arrhythmie des Patienten durch Zuhilfenahme eines EKGs zu erfassen.

18. Vorrichtung nach Anspruch 1 oder Anspruch 2, wobei die Vorrichtung dazu ausgelegt ist, Zeitintervalle ($t$) zwischen den Spitzenwerten des arteriellen Pulsdrucks ($PP$) von einem Puls zum nächsten zu registrieren, einen mittleren Zeitintervallwert ($\bar{t}$) auf der Grundlage des Respirationsveränderungsdiagramms zu bestimmen, und wobei die hämodynamische Variable der normalisierte Pulsdruck ($PPn$) ist, der als

$$PPn = PP \frac{t}{\bar{t}}$$

definiert ist, und der hämodynamische Parameter die arterielle Pulsdruckveränderung ($PPV$) ist.

19. Vorrichtung nach Anspruch 18, wobei die Vorrichtung einen beliebigen arteriellen Katheter zum Messen des arteriellen Pulsdrucks ($PP$) aufweist.

20. Vorrichtung nach den Ansprüchen 18 oder 19, wobei die Vorrichtung dazu ausgelegt ist, die Gleichung

$$PPV = 2 \frac{PPn\max - PPn\min}{PPn\max + PPn\min}$$

zu nutzen, wobei $PPn$max der maximale normalisierte arterielle Pulsdruck ($PPn$) pro künstlichen Atmungszyklus ist, und $PPn$min der minimale normalisierte arterielle Pulsdruck ($PPn$) pro künstlichen Atmungszyklus (30) ist.

21. Vorrichtung nach einem der Ansprüche 1 bis 20, wobei die Vorrichtung dazu ausgelegt ist, für jeden Wert des hämodynamischen Parameters die Feststellung der Eignung hiervon auf der Grundlage der Erfassung von unregelmäßigen Atmungsmustern des Patienten durchzuführen.

22. Vorrichtung nach Anspruch 21, wobei die Vorrichtung dazu ausgelegt ist, unregelmäßige Atmungsmuster des Patienten zu erfassen, indem die Werte des hämodynamischen Parameters registriert werden, und zumindest ein künstliches Atmungszyklusmuster zu erfassen, das aus mindestens drei aufeinander folgenden künstlichen Atmungszyklen besteht, die die Werte des hämodynamischen Parameters enthalten, welche eine vorbestimmte Abweichung voneinander überschreiten, um von der hämodynamischen Analyse die Werte des hämodynamischen Parameters auszuschließen, die diesem künstlichen Atmungszyklusmuster zugeordnet sind.

23. Vorrichtung nach Anspruch 22, wobei die vorbestimmte Abweichung 15% des Mittelwerts der Werte des hämodynamischen Parameters beträgt, die dem künstlichen Atmungszyklusmuster zugeordnet sind.

24. Vorrichtung nach Anspruch 21, wobei die Vorrichtung dazu ausgelegt ist, unregelmäßige Atmungsmuster des Patienten zu erfassen, indem eine Atemwegs-Druckkurve oder eine zentralvenöse Druckkurve oder ein auf den Thorax bezogenes Bioimpedanzsignal oder eine Atemwegs-Strömungskurve, oder eine kapnographische Kurve, oder ein auf die Respiration bezogenes, induktives plethysmographisches Signal oder ein Magnetometersignal oder eine Atemvolumenmessung herangezogen wird.

25. Vorrichtung nach einem der Ansprüche 1 bis 24, wobei die Vorrichtung dazu ausgelegt ist, das Respirationsveränderungsdiagramm der hämodynamischen Variablen derart anzuzeigen, dass das Respirationsveränderungsdiagramm als vertikales Balkenschaubild angezeigt wird, wobei für jeden Einzelpuls-Kurvenabschnitt der hämodynamischen Variablen ein einzelner Balken gezeichnet wird, der zwischen dem Maximalwert und dem Minimalwert des Einzelpuls-Kurvenabschnitts der hämodynamischen Variablen definiert ist.

**Revendications**

1. Appareil pour évaluer un état hémodynamique de patient ventilé mécaniquement, adapté pour analyser une ventilation respiratoire d'une variable hémodynamique, et étant adapté pour dériver la valeur d'un paramètre hémodynamique pour chaque cycle de respiration mécanique
**caractérisé en ce que**
l'appareil est adapté pour dériver une estimation de l'adaptabilité de ladite valeur du paramètre hémodynamique pour l'analyse hémodynamique sur la base d'un diagramme de variation respiratoire.

2. Appareil selon la revendication 1, dans lequel l'appareil est adapté pour fournir un diagramme de variation respiratoire d'une variable hémodynamique.

3. Appareil selon la revendication 1 ou la revendication 2, dans lequel la variable hémodynamique est la pression de pouls artériel (PP) et le paramètre hémodynamique est la variation de pression de pouls artériel (PPV).

4. Appareil selon la revendication 3, dans lequel l'appareil comprend un cathéter artériel pour mesurer la pression de pouls artériel (PP).

5. Appareil selon la revendication 3 ou 4, dans lequel l'appareil est adapté pour utiliser l'équation

$$PPV = 2\frac{PPmax - PPmin}{PPmax + PPmin},$$

dans laquelle PPmax (21) est la pression de pouls artériel (PP) maximum par cycle de respiration mécanique (30) et PPmin (22) est la pression de pouls artériel (PP) minimum par cycle de respiration mécanique (30).

6. Appareil selon la revendication 1 ou la revendication 2, dans lequel la variable hémodynamique est la pression systolique artérielle et le paramètre hémodynamique est la variation de pression systolique artérielle.

7. Appareil selon la revendication 6, dans lequel l'appareil comprend un cathéter artériel pour mesurer la pression systolique artérielle.

8. Appareil selon la revendication 1 ou la revendication 2, dans lequel la variable hémodynamique est le volume systolique cardiaque et le paramètre hémodynamique est la variation de volume systolique.

9. Appareil selon la revendication 8, dans lequel l'appareil comprend un équipement permettant la mesure battement par battement du volume systolique cardiaque.

10. Appareil selon la revendication 1 ou la revendication 2, dans lequel la variable hémodynamique est la courbe d'onde pléthysmographique d'oxymétrie de pouls et le paramètre hémodynamique est la variation de courbe d'onde pléthysmographique d'oxymétrie de pouls.

11. Appareil selon la revendication 10, dans lequel l'appareil comprend une sonde d'oxymètre de pouls pour mesurer la courbe d'onde pléthysmographique d'oxymétrie de pouls.

12. Appareil selon la revendication 1 ou la revendication 2, dans lequel la variable hémodynamique est la période de pré-éjection et le paramètre hémodynamique est la variation de période de pré-éjection.

13. Appareil selon la revendication 12, dans lequel l'appareil comprend un moyen pour enregistrer simultanément l'électrocardiogramme et soit un signal pléthysmographique d'oxymètre de pouls soit un signal de pression artérielle pour déterminer la période de pré-éjection.

14. Appareil selon l'une quelconque des revendications 1 à 13, dans lequel l'appareil est adapté pour réaliser, pour chaque valeur du paramètre hémodynamique, l'estimation de son adaptabilité sur la base de la détection d'une arythmie cardiaque du patient.

15. Appareil selon la revendication 14, dans lequel l'appareil est adapté pour détecter l'arythmie du patient en enregistrant les intervalles temporels entre les crêtes battement à battement de la variable hémodynamique, en déterminant une valeur d'intervalle temporel moyen sur la base du diagramme de variation respiratoire et en détectant un cycle de respiration mécanique comprenant au moins un intervalle temporel excédant une déviation prédéterminée par rapport à la valeur d'intervalle temporel moyen afin d'exclure de l'analyse hémodynamique la valeur du paramètre hémodynamique assigné au cycle de respiration mécanique.

16. Appareil selon la revendication 15, dans lequel la déviation prédéterminée est de 15% de la valeur d'intervalle temporel moyen.

17. Appareil selon la revendication 14, dans lequel l'appareil est adapté pour détecter une arythmie du patient en utilisant un électrocardiogramme.

18. Appareil selon la revendication 1 ou la revendication 2, dans lequel l'appareil est adapté pour enregistrer des intervalles temporels (t) entre les crêtes battement à battement de la pression de pouls artériel (PP), déterminer une valeur d'intervalle temporel moyen ($\bar{t}$) sur la base du diagramme de variation respiratoire, et dans lequel la variable hémodynamique est la pression de pouls normalisée (PPn) définie en tant que

$$PPn = PP\frac{\bar{t}}{t},$$

et le paramètre hémodynamique est la variation de pression de pouls artériel (PPV).

19. Appareil selon la revendication 18, dans lequel l'appareil comprend un cathéter artériel pour mesurer la pression de pouls artériel (PP).

20. Appareil selon la revendication 18 ou 19, dans lequel l'appareil est adapté pour utiliser l'équation

$$PPV = 2\frac{PPn\,max - PPn\,min}{PPn\,max + PPn\,min},$$

dans laquelle PPnmax est la pression de pouls artériel normalisée (PPn) maximum par cycle de respiration mécanique et PPnmin est la pression de pouls artériel normalisée (PPn) minimum par cycle de respiration mécanique (30).

21. Appareil selon l'une quelconque des revendications 1 à 20, dans lequel l'appareil est adapté pour réaliser, pour chaque valeur du paramètre hémodynamique, l'estimation de son adaptabilité sur la base de la détection de motifs de respiration irréguliers du patient.

22. Appareil selon la revendication 21, dans lequel l'appareil est adapté pour détecter des motifs de respiration irréguliers du patient en enregistrant les valeurs du paramètre hémodynamique et en détectant au moins un motif de cycle de respiration mécanique constitué d'au moins trois cycles de respiration mécanique consécutifs comprenant les valeurs du paramètre hémodynamique excédant une déviation prédéterminée par rapport aux autres afin d'exclure de l'analyse hémodynamique les valeurs du paramètre hémodynamique assignées audit motif de cycle de respiration mécanique.

23. Appareil selon la revendication 22, dans lequel la déviation prédéterminée est de 15% de la valeur moyenne des valeurs du paramètre hémodynamique assignées audit motif de cycle de respiration mécanique.

24. Appareil selon la revendication 21, dans lequel l'appareil est adapté pour détecter des motifs de respiration irréguliers du patient en utilisant une courbe de pression des voies aériennes ou une courbe de pression de vaine centrale ou un signal de bio-impédance thoracique ou une courbe de débit des voies aériennes ou une courbe capnographique ou un signal pléthysmographique inductif respiratoire ou un signal de magnétomètre ou une mesure de volume tidal.

25. Appareil selon l'une quelconque des revendications 1 à 24, dans lequel l'appareil est adapté pour afficher le diagramme de variation respiratoire de la variable hémodynamique de telle sorte que le diagramme de variation respiratoire soit représenté en tant que graphique en barres verticales, dans lequel, pour chaque section de courbe de battement à battement de la variable hémodynamique, une barre individuelle est tracée, laquelle est définie entre la valeur maximum et la valeur minimum de la section de courbe de battement à battement de la variable hémodynamique.

Figure 1

Figure 2

Figure 3

Figure 4

$$PPV_1 \neq PPV_2 \neq PPV_3$$

Figure 5

arterial pulse pressure diagram (51-52)
± airway pressure curve (55)
± airway flow curve or tital volume (54)
± ECG or
  thoracic bioimpedance or
  inductive plethysmographic or
  magnetometer signal (58-59)
± central venous pressure curve (56-57)
± capnographic curve (60-61)
± esophageal pressure curve

Figure 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6585658 B

- US 5769082 A

**Non-patent literature cited in the description**

- **BENDJELID.** *J Appl Physiol,* 2004, vol. 96, 337-342